# EUROPEAN PATENT APPLICATION

(11) **EP 4 509 836 A1**
(43) Date of publication of application: **19.02.2025**
(21) Application number: 23788535.5
(22) Date of filing: 07.04.2023
(51) Int. Cl.: G01N 33/52, G01N 33/50, G01N 33/483

(54) **ELEMENT FOR EVALUATING METASTATIC PROPERTIES OF CANCER CELLS, AND METHOD FOR EVALUATING METASTATIC PROPERTIES OF CANCER CELLS**

(30) Priority: 15.04.2022 KR 20220047126
(71) Applicant: KOREA INSTITUTE OF MATERIALS SCIENCE, Gyeongsangnam-do 51508 (KR); Research & Business Foundation SungKyunKwan University, Gyeonggi-do 16419 (KR); Samsung Life Public Welfare Foundation, Seoul 04348 (KR)
(72) Inventor: CHUNG, Kyeong Woon, Daegu 41585 (KR); LEE, Min Young, Changwon-si, Gyeongsangnam-do 51460 (KR)
(74) Representative: dompatent von Kreisler Selting Werner - Partnerschaft von Patent- und Rechtsanwälten mbB
(86) International application number: PCT/KR2023/004690
(87) International publication number: WO 2023/200184

(57) **Abstract**

The present invention provides a device and a method for evaluating the metastatic properties of cancer cells on the basis of cell traction forces of cancer cells.

## Description

### Technical Field

This application claims the benefit of the filing date of Korean Patent Application No. 10-2022-0047126 filed with the Korean Intellectual Property Office on April 15, 2022, the entire contents of which are incorporated in the present invention.

The present invention relates to a device and a method for evaluating the metastatic properties of cancer cells. Specifically, the present invention relates to a device and a method for evaluating the metastatic properties of cancer cells on the basis of the cell traction force of the cancer cells.

### Background Art

Metastasis can be defined as the spread of a tumor from the site where the tumor originated to a different site that is physically separated. Benign tumors are not invasive, and thus do not metastasize, but almost all malignant tumors (cancers) are invasive and can metastasize. The main metastatic pathways of malignant tumors are body surface-body cavity, hematogenous, and lymphatic vessels, and the spread and growth of cancer cells on the body surface and cavity like "sowing seeds" is called direct seeding. Hematogenous metastasis means that cancer cells pass through the blood vessel wall, enter the blood vessel, travel through the bloodstream, arrive at another site, and then grow and divide at that site.

Although cancer metastasis accounts for more than 90% of cancer-related deaths, modern medicine unfortunately still fails to predict cancer metastasis or propose optimized treatments for metastatic cancer patients. Many studies have shown that invasion of cancer cells into blood vessels is necessary for metastasis to other organs, and at this time, the essential mechanism of cancer metastasis, epithelial-mesenchymal transition (EMT), strongly promotes the motility of cancer cells. In addition, EMT has been proven to be a major resistance mechanism against cancer chemotherapy and immunotherapy, and it has been reported that the prognosis of patients showing strong EMT is the worst. Therefore, the clinical need for an accurate and efficient EMT evaluation platform is emerging for the development of cancer treatment technologies, including next-generation cancer immunotherapy. However, currently, there is no clinical evaluation method that can accurately and efficiently evaluate the level of EMT in cancer tissues. Transcriptome analysis, which is a current representative EMT analysis method, is difficult to apply clinically because it requires a lot of time, cost, and expertise for analysis. Even if genes having high relevance with EMT are derived from numerous pathways and signatures, the analysis accuracy for patient samples is low due to the different genomic characteristics of patient's cancer tissues.

As a completely new method for analyzing the metastatic properties and EMT of cancer cells, the point that the present invention focuses on is the correlation between the metastatic properties and EMT of cancer cells and cell traction force. Cells maintain normal tissue structure and function or cause pathological changes in tissues by altering the expression of extracellular matrix genes and proteins. In addition to external forces acting on the cell itself, cells generate their own mechanical force acting on the extracellular matrix, which is called cell traction force (CTF). Through cell traction force, cells can perform various functions such as maintaining cell shape, migrating within tissues, remodeling ECM, and communicating with neighboring cells, and regulate cell functions including DNA synthesis, ECM protein secretion, and cell differentiation by altering the extracellular matrix.

The mechanical signal of cell traction force plays an important role in cell functions such as cell migration, proliferation, and differentiation, and cancer cells lose cell-to-cell adhesion through the EMT process, and gain motility and invasiveness as their binding force with the extracellular matrix increases. Through this process, cancer cells increase the cell traction force they exert on the matrix. In fact, it has been reported that cell traction force significantly increases in EMT-induced cells, and cells with high metastatic potential exhibit higher cell traction force than cells without high metastatic potential.

Various methods have been studied as methods for quantitatively measuring such cell traction force. Recently, a method using cell traction force microscopy has been reported, in which fluorescent microbeads are embedded in a polyacrylamide gel matrix, the displacement of the fluorescent microbeads due to cell traction force when cells are incubated is imaged, and the cell traction force is calculated through data processing on the basis of the movement of the fluorescent beads before and after cell injection. However, this method requires the fabrication of a very sophisticated device, involves image acquisition as well as a complex data processing process considering the physical properties of the gel matrix, and allows analysis of only a very small number of cells in a single device, making it difficult to obtain statistical cell traction force data for cell populations.

In addition, a method has been reported in which micropillars made of PDMS, an elastic material, are fabricated, cells are incubated thereon to allow the cells to attach to the top of the micropillars, and the traction force exerted by the cells is calculated based on the degree of bending of the micropillars due to the cell traction force and the correlation with the physical properties of the micropillars. However, in this method as well, there is a problem in that, since cell traction force should be measured through data processing on the basis of the degree of bending in consideration of the physical properties of the micropillars, this method also requires sophisticated device fabrication and image acquisition/processing, and has difficulty in obtaining statistical data, making it difficult to apply the method to actual clinical settings and drug screening fields.

Accordingly, in order to analyze metastatic properties of cancer cells based on cell traction force, a new approach differentiated from conventional measurement methods is needed.

### DISCLOSURE

### Technical Problem

The technical problem to be solved by the present invention is to provide a device and a method for evaluating the metastatic properties of cancer cells on the basis of the cell traction force of the cancer cells.

However, the problems to be solved by the present invention are not limited to the above-mentioned problems, and other problems not mentioned will be clearly understood by those skilled in the art from the following description.

### Technical Solution

According to one aspect of the present invention, there is provided a device for evaluating the metastatic properties of cancer cells, comprising an organic molecular compound that responds in the form of any one of color change, fluorescence change, luminescence change, and combinations thereof by the cell traction force of metastatic cancer cells.

According to one aspect of the present invention, the device may further comprise, in addition to the organic molecular compound, an extracellular matrix (ECM) polymer.

According to one aspect of the present invention, the organic molecular compound may be a diketopyrrolopyrrole-based compound containing a straight- or branched-chain alkyl group having 5 to 10 carbon atoms and a halogen group.

According to one aspect of the present invention, the device for evaluating the metastatic properties of cancer cells may comprise one or more of: a power supply unit configured to control power supply; a cell incubation unit configured to incubate cancer cells; an input unit configured to apply cancer cells; an output unit comprising the organic molecular compound, or the organic molecular compound and the extracellular matrix polymer, and configured to output the response of the organic molecular compound, which is exhibited by the cell traction force of metastatic cancer cells; an amplification unit configured to amplify the response from the output unit, which is in the form of any one of color change, fluorescence change, luminescence change, and combinations thereof, and provide visual information that may be confirmed with the naked eye or an instrument; and, when there are two or more output units, a result unit configured to combine the responses from the output units and display numerical data.

According to one aspect of the present invention, when the device for evaluating the metastatic properties of cancer cells comprises a plurality of output units, the plurality of output units may each independently comprise different types of extracellular matrix polymers.

According to one aspect of the present invention, when the device for evaluating the metastatic properties of cancer cells comprises a plurality of output units, the plurality of output units may each independently comprise different concentrations of the extracellular matrix polymer.

According to one aspect of the present invention, when the device for evaluating the metastatic properties of cancer cells comprises a plurality of output units, the plurality of output units may each independently output responses.

According to one aspect of the present invention, there is provided a method for evaluating the metastatic properties of cancer cells using the device for evaluating the metastatic properties of cancer cells.

According to one aspect of the present invention, there is provided a method for evaluating the metastatic properties of cancer cells, comprising steps of: applying cancer cells to be evaluated onto an organic molecular compound; and determining that the cancer cells are metastatic cancer cells, if a response of the organic molecular compound, which appears in the form of any one of color change, fluorescence change, luminescence change, and combinations thereof, is detected.

### Advantageous Effects

The device for evaluating the metastatic properties of cancer cells according to one embodiment of the present invention may statistically evaluate the metastatic potential of a large number of cancer cells simply and efficiently in evaluating the metastatic potential of cancer cells.

The method for evaluating the metastatic properties of cancer cells according to one embodiment of the present invention may statistically evaluate the metastatic potential of a large number of cancer cells simply and efficiently.

The effects of the present invention are not limited to the above-described effects, and other effects not mentioned will be clearly understood by those skilled in the art from the present specification.

### Brief Description of Drawings

FIG. 1 shows the results of applying gastric cancer cell line MKN-1 and ovarian cancer cell line SKOV-3 to the devices for evaluating the metastatic properties of cancer cells, fabricated in Examples 2 and 9.
FIG. 2 shows the results of applying gastric cancer cell line MKN-1 to the devices for evaluating the metastatic properties of cancer cells, fabricated in Examples 1 and 4 to 8.
FIG. 3a shows the results of applying gastric cancer cell line MKN-1 and gastric cancer cell line MKN-45 to the devices for evaluating the metastatic properties of cancer cells, fabricated in Examples 1 to 3.
FIG. 3b shows the results of applying ovarian cancer cell line SKOV-3 or ovarian cancer cell line OVCAR-3 to the devices for evaluating the metastatic properties of cancer cells, fabricated in Examples 6, 9, and 10.
FIG. 4 shows the results of applying gastric cancer cell line MKN-1 and gastric cancer cell line MKN-45 to the device for evaluating the metastatic properties of cancer cells, fabricated in Example 1.

### Best Mode

Throughout the present specification, it is to be understood that when any part is referred to as "comprising" any component, it does not exclude other components, but may further comprise other components, unless otherwise specified.

Throughout the present specification, when any member is referred to as being "on" another member, it not only refers to a case where any member is in contact with another member, but also a case where a third member exists between the two members.

Hereinafter, the present invention will be described in more detail.

According to one embodiment of the present invention, there is provided a device for evaluating the metastatic properties of cancer cells, comprising an organic molecular compound that responds in the form of any one of color change, fluorescence change, luminescence change, and combinations thereof by the cell traction force of metastatic cancer cells.

The device for evaluating the metastatic properties of cancer cells according to one embodiment of the present invention may efficiently and statistically diagnose the metastatic potential of malignant tumors (i.e., cancer), that is, the possibility of metastasis, at an early stage, and evaluate the metastatic properties of cancer cells, by comprising an organic molecular compound that responds in the form of any one of color change, fluorescence change, luminescence change, and combinations thereof by detecting the cell traction force of metastatic cancer cells, which corresponds to a very small physical stimulus.

Specifically, the structure or arrangement of the compound is changed very sensitively by a physical stimulus, and accordingly, the use of the organic molecular compound exhibiting a very large fluorescence change makes it possible to distinguish highly metastatic cancer cells from less metastatic cancer cells on the basis of the presence or absence of responses such as color change, fluorescence change, and luminescence change.

According to one embodiment of the present invention, the "color change" may mean that the color exhibited by reflection or transmission of visible light from the compound changes. For example, a red organic molecular compound may be changed to blue by the cell traction force of metastatic cancer cells.

According to one embodiment of the present invention, the "fluorescence change" may mean a change in fluorescence color and/or fluorescence intensity, and fluorescence refers to a phenomenon in which external light energy is absorbed and emitted as light. For example, an organic molecular compound with dark green fluorescence may be changed to bright green fluorescence by the cell traction force of metastatic cancer cells (change in fluorescence intensity).

According to one embodiment of the present invention, the "luminescence change" may mean that a compound that did not emit light by itself emits light by an external stimulus. For example, an organic molecular compound that did not emit light may emit light by the cell traction force of metastatic cancer cells.

According to one embodiment of the present invention, the organic molecular compound is not particularly limited, and may be selected from among compounds, such as mechanochromic materials, piezochromic materials, tribochromic materials, etc., which respond in the form of any one of color change, fluorescence change, luminescence change, and combinations thereof to physical stimuli.

When the compound that responds in the form of any one of color change, fluorescence change, luminescence change, and combinations thereof as described above is used, it is possible to distinguish highly metastatic cancer cells from less metastatic cancer cells on the basis of the color change, fluorescence change, and luminescence change of the organic compound by the cell traction force with the naked eye or an instrument. In other words, in less metastatic cancer cells, no change is observed, but in highly metastatic cancer cells, the metastatic potential of the target cancer cells may be determined based on distinct color change, fluorescence change and/or luminescence change.

According to one embodiment of the present invention, the organic molecular compound may be a diketopyrrolopyrrole-based compound containing a straight- or branched-chain alkyl group having 5 to 10 carbon atoms and a halogen group. Specifically, the organic molecular compound may be a diketopyrrolopyrrole-based compound containing an octyl group and a bromine (Br) group.

The device for evaluating the metastatic properties of cancer cells according to one embodiment of the present invention may further comprise an extracellular matrix (ECM) polymer that interacts with the organic molecular compound. The extracellular matrix polymer may serve to allow cells to adhere well, thereby allowing the cell traction force to be better transmitted to the organic molecular compound, thus improving the sensitivity of the organic molecular compound. Any polymer to which cells adhere well may be used without particular limitation, and for example, a polymer such as collagen, fibronectin, laminin, vitronectin, etc. may be used.

According to one embodiment of the present invention, the organic molecular compound and the extracellular matrix (ECM) polymer may be used in a specific combination. Specifically, the organic molecular compound may be used in combination with, in particular, an extracellular matrix polymer that can enhance the sensitivity to cell traction force, among various extracellular matrix polymers.

According to one embodiment of the present invention, the device for evaluating the metastatic properties of cancer cells may comprise one or more of: a power supply unit configured to control power supply; a cell incubation unit configured to incubate cancer cells; an input unit configured to apply cancer cells; an output unit comprising the organic molecular compound, or the organic molecular compound and the extracellular matrix polymer, and configured to output the response of the organic molecular compound, which is exhibited by the cell traction force of metastatic cancer cells; an amplification unit configured to amplify the response from the output unit, which is in the form of any one of color change, fluorescence change, luminescence change, and any combination thereof, and provide visual information that may be confirmed with the naked eye or an instrument; and, when there are two or more output units, a result unit configured to combine the responses from the output units and display numerical data.

According to one embodiment of the present invention, the power supply unit may be provided when power supply is required, and such a power supply unit may be in the form of being connected to an external power source or in the form of having a built-in battery.

According to one embodiment of the present invention, the cell incubation unit may be provided to incubate cancer cells, and it may be provided when cell incubation is necessary to obtain a degree of cells required for measurement from a small amount of cancer cells.

According to one embodiment of the present invention, the input unit corresponds to a part to which cancer cells are applied, and cancer cells may be injected into, applied to, or incubated in the input unit.

According to one embodiment of the present invention, the output unit may comprise the organic molecular compound, or comprise the organic molecular compound and the extracellular matrix polymer. The output unit may respond to the cell traction force of the cancer cells in the input unit as a physical stimulus and output the response in the form of any one of color change, fluorescence change, luminescence change, and combinations thereof.

According to one embodiment of the present invention, when the device for evaluating the metastatic properties of cancer cells comprises a plurality of output units, the plurality of output units may each independently comprise different types of extracellular matrix polymers, or may each independently comprise different concentrations of the extracellular matrix polymer.

According to one embodiment of the present invention, when the device for evaluating the metastatic properties of cancer cells comprises a plurality of output units, the plurality of output units may each independently output the responses. The output units may have different degrees of sensitivity to the cell traction force. As described above, even if the output units comprise different types of extracellular matrix polymer or comprise the same organic molecular compound and extracellular matrix polymer, the output units may have different sensitivities because they have different concentrations of the extracellular matrix polymer. Accordingly, the reference stimulus at which the response is output may be different for each output unit, and thus the accuracy of evaluating the metastatic properties of cancer cells may be higher.

According to one embodiment of the present invention, the output unit may have a multilayer structure comprising a first layer and a second layer. The first layer may be formed by coating with a melt or solution containing the organic molecular compound. The solution may contain an organic solvent, and specifically, the coating may be performed by a drop casting, blading, or spin coating method.

In addition, the second layer may be formed by coating the first layer with an aqueous solution containing the extracellular matrix polymer.

According to one embodiment of the present invention, the input unit and the output unit may be configured as one part. That is, the device for evaluating the metastatic properties of cancer cells according to one embodiment of the present invention may comprise an input/output unit. The input/output unit may be a part where cancer cells are directly applied to the organic molecular compound, and input and output may be performed in the same part.

According to one embodiment of the present invention, the amplification unit may be provided to amplify the response from the output unit. The cell traction force of a single cell and the response of the compound thereto may be very small, and the amplification unit may be provided when it is necessary to amplify such a response to enable measurement.

According to one embodiment of the present invention, the result unit may combine and display the responses from a plurality of output units. Specifically, when there are two or more output units, the result unit may statistically process the response from each output unit and display the metastatic properties of cancer cells.

According to one embodiment of the present invention, there is provided a method for evaluating the metastatic properties of cancer cells using the device for evaluating the metastatic properties of cancer cells.

According to one embodiment of the present invention, there is provided a method for evaluating the metastatic properties of cancer cells, comprising steps of: applying cancer cells to be evaluated onto an organic molecular compound; and determining that the cancer cells are metastatic cancer cells, if a response of the organic molecular compound, which appears in the form of any one of color change, fluorescence change, luminescence change, and combinations thereof, is detected.

According to one embodiment of the present invention, after cancer cells to be evaluated are applied onto an organic molecular compound, if the response that appears in the form of any one of color change, fluorescence change, luminescence change, and combinations thereof is not detected, the cancer cells may be determined to be less metastatic, and if the reaction is detected, the cancer cells may be determined to be highly metastatic cancer cells.

According to one embodiment of the present invention, after the step of applying cancer cells to be evaluated onto an organic molecular compound, it may be checked whether the response is detected immediately after the step or after 7 days.

According to one embodiment of the present invention, the type of cancer cells to be evaluated is not limited. For example, the cancer cells may be gastric cancer cells, lung cancer cells, breast cancer cells, ovarian cancer cells, colorectal cancer cells, etc.

### Mode for Invention

Hereinafter, the present invention will be described in detail by way of examples. However, the examples according to the present invention may be modified in various different forms, and the scope of the present invention should not be construed as being limited to the examples described below. The examples in the present specification are provided to more fully explain the present invention to those skilled in the art.

### Example 1

A film was formed on a substrate using a solution containing a diketopyrrolopyrrole compound having the structure of the Formula below by a spin coating method. The coating layer was additionally coated with an aqueous solution containing 200 µg/ml of collagen II as an extracellular matrix polymer by a drop casting method, followed by washing three times or more with distilled water, thereby fabricating a device for evaluating the metastatic properties of cancer cells.

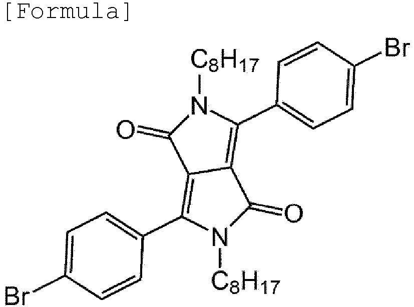

### Example 2

A device for evaluating the metastatic properties of cancer cells was fabricated in the same manner as in Example 1, except that the coating layer was coated with an aqueous solution containing 100 µg/ml of collagen II as an extracellular matrix polymer.

### Example 3

A device for evaluating the metastatic properties of cancer cells was fabricated in the same manner as in Example 1, except that the coating layer was coated with an aqueous solution containing 50 µg/ml of collagen II as an extracellular matrix polymer.

### Example 4

A device for evaluating the metastatic properties of cancer cells was fabricated in the same manner as in Example 1, except that collagen I was used instead of collagen II.

### Example 5

A device for evaluating the metastatic properties of cancer cells was fabricated in the same manner as in Example 1, except that collagen IV was used instead of collagen II.

### Example 6

A device for evaluating the metastatic properties of cancer cells was fabricated in the same manner as in Example 1, except that fibronectin was used instead of collagen II.

### Example 7

A device for evaluating the metastatic properties of cancer cells was fabricated in the same manner as in Example 1, except that laminin was used instead of collagen II.

### Example 8

A device for evaluating the metastatic properties of cancer cells was fabricated in the same manner as in Example 1, except that vitronectin was used instead of collagen II.

### Example 9

A device for evaluating the metastatic properties of cancer cells was fabricated in the same manner as in Example 1, except that the coating layer was coated with an aqueous solution containing 100 µg/ml of fibronectin as an extracellular matrix polymer by a drop casting method.

### Example 10

A device for evaluating the metastatic properties of cancer cells was fabricated in the same manner as in Example 1, except that the coating layer was coated with an aqueous solution containing 50 µg/ml of fibronectin as an extracellular matrix polymer by a drop casting method.

### Experimental Example 1: Evaluation Depending on Cell Attachment Period

Highly metastatic (high EMT) MKN-1 gastric cancer cell line and highly metastatic (high EMT) SKOV-3 ovarian cancer cell line were each applied to the devices for evaluating the metastatic properties of cancer cells, fabricated in Examples 2 and 9, and the fluorescence signals of the devices were observed after 1 day, 2 days, or 3 days.

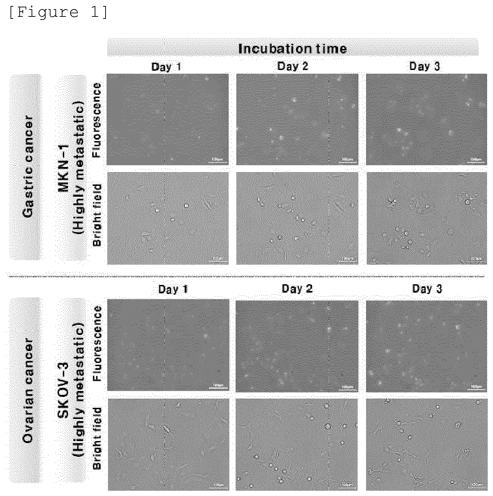

FIG. 1 shows bright field images representing the actual cell morphology and fluorescence images representing the fluorescence signals of the devices, obtained by microscopy after applying the gastric cancer cell line MKN-1 and the ovarian cancer cell line SKOV-3 to the devices for evaluating the metastatic properties of cancer cells, fabricated in Examples 2 and 9, respectively, and incubating the cell lines for 1 day, 2 days, or 3 days.

Observations were made through the Olympus IX-83 fluorescence microscope, and the bright field images were observed at 10x magnification using Transmitted DIC Mirror Unit IX3-FDICT, and the fluorescence was observed at 10× magnification using excitation filter 460-495, dichroic mirror DM505, interference barrier filter 510IF, power 25%, and exposure time 200 ms. The same applies hereinafter.

Referring to FIG. 1, it can be confirmed that, from 1 day onwards, green-yellow fluorescence changes (turn-on fluorescence signals) started to be observed due to the crystallization of the diketopyrrolopyrrole compound by cell traction forces, and in the case of the devices after 2 days, distinct fluorescence changes due to cell traction forces were observed.

### Experimental Example 2: Optimization Depending on Type of Extracellular Matrix Polymer

Highly metastatic (high EMT) MKN-1 gastric cancer cell line was applied to each of the devices for evaluating the metastatic properties of cancer cells, fabricated in Examples 1 and 4 to 8, and the fluorescence signals of the devices were observed after 2 days.

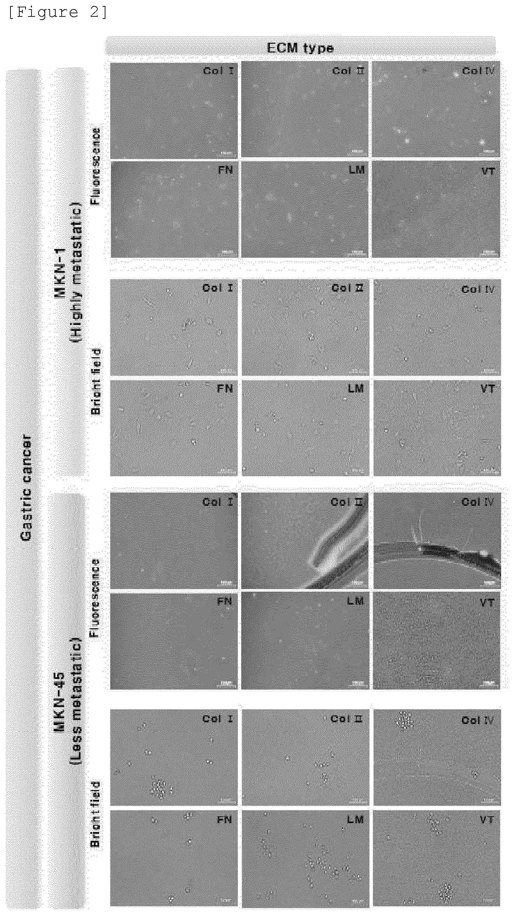

FIG. 2 shows bright field images representing the actual cell morphology and fluorescence images representing the fluorescence signals of the devices, obtained by microscopy after applying the gastric cancer cell line MKN-1 to the devices for evaluating the metastatic properties of cancer cells, fabricated in Examples 1 and 4 to 8, and incubating the cell line for 2 days.

Referring to FIG. 2, highly metastatic MKN-1 can be confirmed in all of Examples 1 and 4 to 8 through fluorescence changes.

### Experimental Example 3: Observation and Evaluation of Responses Depending on Extracellular Matrix Polymer Concentration

Highly metastatic (high EMT) MKN-1 gastric cancer cell line and less metastatic (low EMT) MKN-45 gastric cancer cell line were each applied to the devices for evaluating the metastatic properties of cancer cells, fabricated in Examples 1 to 3, and the fluorescence signals of the devices were observed after 2 days.

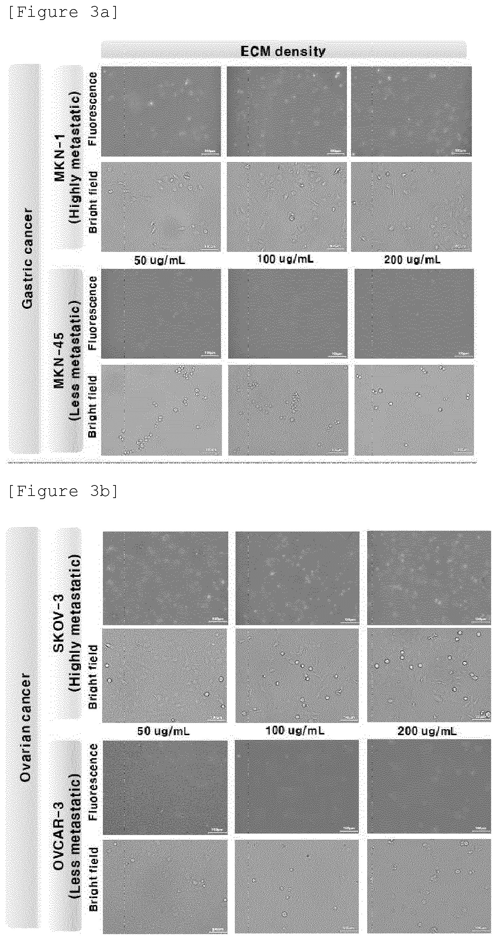

FIG. 3a shows bright field images representing the actual cell morphology and fluorescence images representing the fluorescence signals of the devices, obtained by microscopy after applying the gastric cancer cell line MKN-1 or the gastric cancer cell line MKN-45 to the devices for evaluating the metastatic properties of cancer cells, fabricated in Examples 1 to 3, and incubating the cell line for 2 days.

Referring to FIG. 3a, for highly metastatic MKN-1, the fluorescence change increased with increasing extracellular matrix polymer concentration, whereas for less metastatic MKN-45, no fluorescence change was observed in all the devices of the Examples regardless of the change in extracellular matrix polymer concentration.

In addition, highly metastatic (high EMT) SKOV-3 ovarian cancer cell line and less metastatic (low EMT) OVCAR-3 ovarian cancer cell line were each applied to the devices for evaluating the metastatic properties of cancer cells, fabricated in Examples 6, 9, and 10, and the fluorescence signals of the devices were observed after 2 days.

FIG. 3b shows bright field images representing the actual cell morphology and fluorescence result images representing the fluorescence signals of the devices, obtained by microscopy after applying the ovarian cancer cell line SKOV-3 or the ovarian cancer cell line OVCAR-3 to the devices for evaluating the metastatic properties of cancer cells, fabricated in Examples 6, 9, and 10, and incubating the cell line for 2 days.

Referring to FIG. 3b, it was confirmed that, similar to the evaluation results for MKN-1 in FIG. 3a, the degree of fluorescence change increased with increasing concentration of the extracellular matrix polymer for the highly metastatic ovarian cancer cell line SKOV-3. Meanwhile, in the case of less metastatic OVCAR-3, no fluorescence change was observed in the devices of Examples 9 and 10 with low fibronectin concentration, but fluorescence signals were observed in some cells in the device of Example 6.

The results in FIGS. 3a and 3b are consistent with previous reports that cell traction force increases with increasing concentration of the extracellular matrix polymer, and it can be confirmed that the patterns of fluorescence changes depending on the concentration of the extracellular matrix polymer are different between cells showing different metastatic potentials. Thereby, it can be confirmed that it is possible to subdivide the results in the case of having a plurality of output units by adjusting the concentration of the extracellular matrix polymer.

### Example 4: Evaluation of Metastatic Potential of Cancer

Highly metastatic (high EMT) MKN-1 gastric cancer cell line and less metastatic (low EMT) MKN-45 gastric cancer cell line were each applied to the device for evaluating the metastatic properties of cancer cells, fabricated in Example 1, and the fluorescence signals of the device were observed after 2 days.

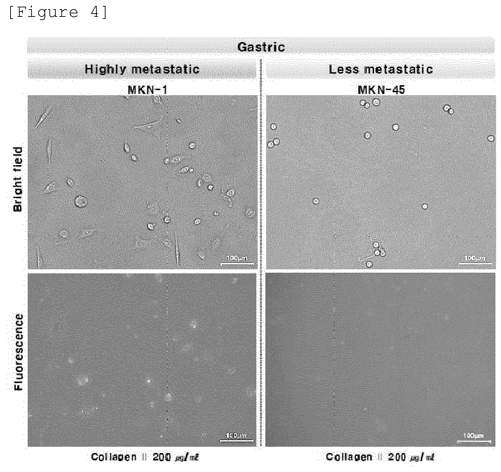

FIG. 4 shows bright field images representing the actual cell morphology and fluorescence images representing the fluorescence signals of the device, obtained by microscopy after applying the gastric cancer cell line MKN-1 and the gastric cancer cell line MKN-45 to the device for evaluating the metastatic properties of cancer cells, fabricated in Example 1, and incubating the cell lines for 2 days.

Referring to FIG. 4, it can be confirmed that, a distinct yellow fluorescence signal was observed in the case in which highly metastatic MKN-1 was applied, whereas only the initial orange fluorescence signal was observed in MKN-45, suggesting the high metastatic potential of MKN-1 and the low metastatic potential of MKN-45.

Considering the well-known facts that MKN-1 is a highly metastatic cancer cell line (high EMT) and MKN-45 is a less metastatic cancer cell line (low EMT), it can be confirmed that the device for evaluating the metastatic properties of cancer cells according to an embodiment of the present invention can effectively and simply evaluate the metastatic potential of cancer cells.

Although the present invention has been described with reference to limited embodiments, the present invention is not limited by these embodiments, and it will be apparent to those skilled in the art to which the present invention pertains that various modifications and variations are possible within the technical spirit of the present invention and the equivalent scope of the claims to be described below.

## Claims

1. A device for evaluating metastatic properties of cancer cells, comprising an organic molecular compound that responds in the form of any one of color change, fluorescence change, luminescence change, and combinations thereof by a cell traction force of metastatic cancer cells.

2. The device according to claim 1, further comprising, in addition to the organic molecular compound, an extracellular matrix (ECM) polymer.

3. The device according to claim 1, wherein the organic molecular compound is a diketopyrrolopyrrole-based compound containing a straight- or branched-chain alkyl group having 5 to 10 carbon atoms and a halogen group.

4. The device according to claim 1, wherein the device comprises one or more of:
a power supply unit configured to control power supply;
a cell incubation unit configured to incubate cancer cells;
an input unit configured to apply cancer cells;
an output unit comprising the organic molecular compound, or the organic molecular compound and an extracellular matrix polymer, and configured to output the response of the organic molecular compound, which is exhibited by the cell traction force of metastatic cancer cells;
an amplification unit configured to amplify the response from the output unit, which is in the form of any one of color change, fluorescence change, luminescence change, and combinations thereof, and provide visual information that is confirmed with the naked eye or an instrument; and,
when there are two or more output units, a result unit configured to combine the responses from the output units and show numerical data.

5. The device according to claim 4, wherein, when the device comprises a plurality of output units, the plurality of output units each independently comprise different types of extracellular matrix polymers.

6. The device according to claim 4, wherein, when the device comprises a plurality of output units, the plurality of output units each independently comprise different concentrations of the extracellular matrix polymer.

7. The device according to any one of claims 5 and 6, wherein, when the device comprises a plurality of output units, the plurality of output units each independently output responses.

8. A method for evaluating metastatic properties of cancer cells using the device according to any one of claims 1 to 7.

9. A method for evaluating metastatic properties of cancer cells, comprising steps of:
applying cancer cells to be evaluated onto an organic molecular compound; and
determining that the cancer cells are metastatic cancer cells, if a response of the organic molecular compound, which appears in the form of any one of color change, fluorescence change, luminescence change, and combinations thereof, is detected.
